# EUROPEAN PATENT SPECIFICATION

(11) **EP 3 984 502 B1**
(45) Date of publication and mention of the grant of the patent: **01.10.2025**
(21) Application number: 21202282.6
(22) Date of filing: 12.10.2021
(51) Int. Cl.: A61F 2/36, A61B 17/16, A61F 2/30

(54) **SELF-BROACHING NECK PRESERVING HIP STEM**
SELBSTSCHNEIDENDER HALSSCHONENDER HÜFTSCHAFT
TIGE DE HANCHE PRÉSERVANT LE COU RÉALISANT LEUR PROPRE ALÉSAGE

(30) Priority: 16.10.2020 US 202063092547 P
(43) Date of publication of application: 20.04.2022
(73) Proprietor: Howmedica Osteonics Corp., Mahwah, NJ 07430 (US)
(72) Inventor: ROY, Shammodip, WAYNE, 07470 (US); SCHOLL, Daniel Rudolf, ANDOVER, 07821 (US); SAUNDERS, Tyler, PARK RIDGE, 07656 (US)
(74) Representative: Regimbeau

(56) References cited:
- EP-A1- 0 208 578
- EP-A1- 0 852 931
- EP-A1- 2 253 291
- WO-A1-2012/072111
- WO-A1-83/02555
- US-A- 5 108 453
- US-A- 5 665 091
- US-A1- 2012 130 502

## Description

### CROSS-REFERENCE TO RELATED APPLICATIONS

This application claims the benefit of the filing date of U.S. Provisional Application No. 63/092,547 filed October 16, 2020.

### BACKGROUND OF THE INVENTION

Patients often require a total hip arthroplasty in the event of arthritis, instability, joint pain, femoral fracture, and the like that a patient may experience. A typical hip prosthesis used in a total hip arthroplasty includes a stem which functions as the base for proximal head replacement of the femur. Stems generally require a resection of the femur in preparation for implantation. With metaphyseal/diaphyseal engaging stems, a surgeon would typically resect a significant amount of good quality healthy bone stock to fit the stem into the femoral canal. Due to the loss of such healthy bone stock, press-fitted cement-less stems are inserted deeper into the canal than may be required otherwise in order to generate adequate fixation and stability. In this regard, conventional stems load the femur relatively distally, and therefore extend deep into the femoral canal to achieve sufficient initial stability and long-term fixation in the metaphyseal region. Consequently, the conventional stem often requires significant preparation of narrow femoral canals up to depths very often exceeding 100 millimeters. Such preparation typically involves extensive instrumentation, such as a family of sequentially increasing sizes of broaches, to prepare the femoral canal up to the required depth. Such instrumentation not only leads to excess equipment used in surgery, but may also lead to decreased stability due to repetitive insertion and removal of broaches of increasing size. Further, in order to be able to present such instruments to the femoral canal, the standard level of resection is distal of the femoral neck such that it leaves little to no bone remaining at the femoral neck. Therefore, further improvements are desirable. EP 2 253 291 A1 discloses a bone implant with a surface anchoring structure, and US 5,108,453 A discloses a bone implant for insertion in a prepared recess of a human joint.

### BRIEF SUMMARY OF THE INVENTION

The invention is defined in independent claim 1.

The present disclosure describes a hip prosthesis having a curved, tapering stem including rasp teeth on a distal-lateral surface capable of self-broaching upon implantation. The stem may extend into the canal slightly past the level of the lesser trochanter, having a length relatively shorter than the conventional tapered wedge and other anatomic stems. The disclosed stem provides more anatomic loading on the medial calcar region of the proximal femur and promotes preservation of bone stock in the femoral neck during a total hip arthroplasty. The implant as described herein provides several benefits that improve the outcome of the procedure, such as a reduced moment arm, improved implant load distribution via greater anatomic loading on the implant, reduced potential for trochanteric fractures, greater head offset and increased joint stability, improved bone remodeling due to less bone resorption in the proximal femur, potential for reduced blood loss, greater preservation of soft tissue in the trochanteric region and potential to reduce thigh pain and improve the timing and experience of rehabilitation and recovery for the patient. Another potential benefit is that a greater amount of bone preserved in the neck may enable easier revisions for patients in the event of failure because there remains good bone stock proximal to the conventional resection level, which can subsequently be removed and revised with a conventional stem.

Preparation of the neck region of the femoral canal proximal to the level of the lesser trochanter may be performed robotically (*e*.*g*., with a rotary cutting tool such as a burr that removes cancellous bone) based on a CT-based 3D plan of the patient, which would enable initial stem insertion into the canal. The stem is intended to be self-broaching to simplify the surgical workflow. In other words, the stem itself can carve its own path in the bone and may not require initial shaping of the canal with other instruments, such as an associated family of sequentially increasing sizes of broaches. This may be achieved with the help of the proximal robotic preparation as well as one or more cutting rasp teeth features disposed on a relief surface on the distal-lateral portion of the stem, as will be discussed below in greater detail. For some patients, preparation of the neck region of the femoral canal may include the formation of a pilot hole using an instrument such as a curved awl, which is also discussed below in greater detail.

The present invention relates to a femoral implant, as defined in the claims. Any of the following "examples" (or methods) listed here that do not fall under the scope of the claims are merely exemplary.

In a first example, a femoral implant may include a spherical head positioned a proximal end of the implant. The femoral implant may further include a neck coupled to the head, the neck extending distally from the head. The femoral implant may further include a stem extending distally from the neck. The stem may have a medial side adapted to face a medial extent of the femur and a lateral side adapted to face a lateral extent of the femur when implanted therein. The medial side may have a concave curvature and the lateral side may have a convex curvature. The stem may include a plurality of teeth disposed on a distal portion of the lateral side of the stem such that the stem is configured to be self-broaching when implanted into the femur. In a second example, the plurality of teeth of the first example may be arranged in a plurality of columns on the lateral side. In a third example, the plurality of teeth of any of the first or second examples may be arranged in an array forming straight rows and columns of teeth on the lateral side, the teeth spaced equally apart from one another. In a fourth example, the plurality of teeth of any of the first through third examples may be arranged on the lateral side in an array having a plurality of rows wherein the teeth of every other row are aligned in columns and the teeth of adj acent rows are offset.

In a fifth example of the first example, the stem of any of the first through fourth examples may include a flange between the distal end of the neck and the proximal end of the stem, the flange having a larger cross-section than the neck defining a peripheral lip. In a sixth example, the proximal portion of the stem of any of the first through fifth examples may include a porous structure. In a seventh example, the porous structure of the sixth example may be positioned more proximally on the lateral side of the proximal stem portion than the medial side of the proximal stem portion. In an eighth example, the medial side of any of the sixth or seventh examples may have a transition point where the medial side changes from the first radius of curvature to the second radius of curvature, and the stem may define a transition plane between the porous structure and a non-porous portion of the stem, wherein the transition point may be located proximal to the transition plane. In a ninth example, the stem of any one of the first through eighth examples may include an anterior side adapted to face an anterior extent of the femur and a posterior side adapted to face a posterior extent of the femur, and the stem may taper in both the medial-lateral direction and the anterior-posterior direction as the stem extends distally. In a tenth example, the plurality of teeth of any one of the first through ninth examples may be disposed on a relief surface located on the lateral side of the stem. The relief surface may extend toward a central axis of the stem in a distal direction. The relief surface may intersect the convex curvature of the stem. In an eleventh example, the relief surface of the tenth example may be located at a relief portion of the stem. The relief portion of the stem may have a polygonal cross section. In a twelfth example, a distal portion of the stem of any one of the first through eleventh examples may be polished. In a thirteenth example, the concave curvature of the stem of any one of the first through twelfth examples may extend from a proximal extent of the stem to a distal extent of the stem. In a fourteenth example, the neck and stem of any one of the first through thirteenth examples may be monolithic, and the spherical head may be a modular piece coupleable to the neck and stem. In a fifteenth example, the medial side of the stem of any one of the first through fourteenth examples may define a first radius of curvature on a proximal portion of the medial side of the stem. The medial side may further define a second radius of curvature on a distal portion of the medial side of the stem. In a sixteenth example, the lateral side of the stem of the fifteenth example may define a third radius of curvature. In a seventeenth example, the second and third radii of curvature of the sixteenth example may be different than the first radius of curvature.

In another first example, a femoral component may include a spherical head positioned at a proximal end of the implant. The femoral implant may further include a neck coupled to the head. The neck may extend distally from the head. The femoral implant may further include a stem extending distally from the neck. The stem may have a medial side adapted to face a medial extent of the femur and a lateral side adapted to face a lateral extent of the femur. The medial side may have a concave curvature and the lateral side may have a convex curvature. The concave curvature may be defined by a first and a second radius of curvature and the convex curvatures may be defined by a third radius of curvature. The stem may further include a relief surface intersecting the convex curvature and extending distally therefrom. The relief surface may have a plurality of teeth such that the stem is configured to be self-broaching when implanted into the femur.

In a further first example, a method of implanting a femoral prosthesis may include resecting a femur through a neck thereof between a head and greater trochanter of the femur such that a portion of the neck is preserved; impacting a femoral implant into the femur, the femoral implant including a stem having a porous bone-ingrowth portion and a convex lateral side that is defined by a radius of curvature and a plurality of teeth disposed on a distal portion of the lateral side ; and cutting the bone using the plurality of teeth which is performed concurrently with the impacting step. In a second example, the method of the first example may further include the step of planning the implantation using a CT-based 3D plan of a patient. In a third example, the resecting step of any one of the first or second steps may include the use of a burr to resect the femur. In a fourth example, the resecting step of any one of the first through third steps may be performed robotically. In a fifth example, the method of any one of the first through fourth examples may further include creating a pilot hole that includes the use of a curved entry instrument to form the hole. In a sixth example, the impacting step of any one of the first through fifth examples may include impacting the implant until the implant forms a press-fit with the resected portion of the femur. In a seventh example, the impacting step of any one of the first through sixth examples may include impacting the implant until a flange disposed at the proximal end of the stem is on or within 2 millimeters of the resection level of the femur.

In still a further first example, a method of implanting a femoral implant may include planning procedure resection location on a femoral neck using a 3D CT scan of a patient; removing a proximal portion of the femoral neck using a robotically controlled high-speed burr so as to leave a distal portion of the femoral neck; preparing a pilot hole that extends into the femoral canal using a curved instrument; and impacting a femoral implant into the pilot hole, wherein the implant includes a plurality of teeth disposed on the distal-lateral surface of the implant to allow for self-broaching. In a second example, the step of removing a proximal portion of the femoral neck of the first example may include robotically tracking the removal of the bone to ensure the bone removal accords with the surgical plan.

### BRIEF DESCRIPTION OF THE DRAWINGS

FIG. 1A is a perspective view of a hip implant according to an embodiment of the disclosure.
FIG. 1B is a schematic view of the hip implant of FIG. 1A illustrating radii of curvature thereof.
FIG. 1C is a close-up view of a distal portion of the hip implant of FIG. 1A.
FIG. 2 is a side view of the hip implant of FIG. 1A.
FIG. 3 is a front view of a solid model of a proximal portion of a femur.
FIG. 4A is a schematic cross-sectional view of a femur receiving the implant of FIG. 1A.
FIG. 4B is a close-up view of the distal portion of the implant in FIG. 4A disposed in the femur.
FIG. 5 is a schematic cross-sectional view of a femur with the implant of FIG. 1A fully implanted.
FIG. 6 is a front view of a hip stem implanted in a femur, and which is disclosed for illustrative purposes only.
FIG. 7 is a superior view of the hip of FIG. 1A implanted in the femur of FIG. 6, and which is disclosed for illustrative purposes only.
FIG. 8 is a perspective view of a curved entry instrument which is disclosed for illustrative purposes only.

### DETAILED DESCRIPTION

The present disclosure will now be described more fully hereinafter with reference to the accompanying drawings, in which some, but not all aspects of the disclosure are shown. Indeed, the disclosure may be embodied in many different forms and should not be construed as being limited to the aspects set forth herein. Like numbers refer to like elements throughout.

As used herein, the term "proximal," when used in connection with a device or components of a device, refers to the end of the device closer to the surgeon or user when the device is being used and implanted as intended. On the other hand, the term "distal," when used in connection with a device or components of a device, refers to the end of the device farther away from the surgeon or user when the device is being used and implanted as intended. As used herein, the terms "substantially," "generally," "approximately," and "about" are intended to mean that slight deviations from absolute are included within the scope of the term so modified.

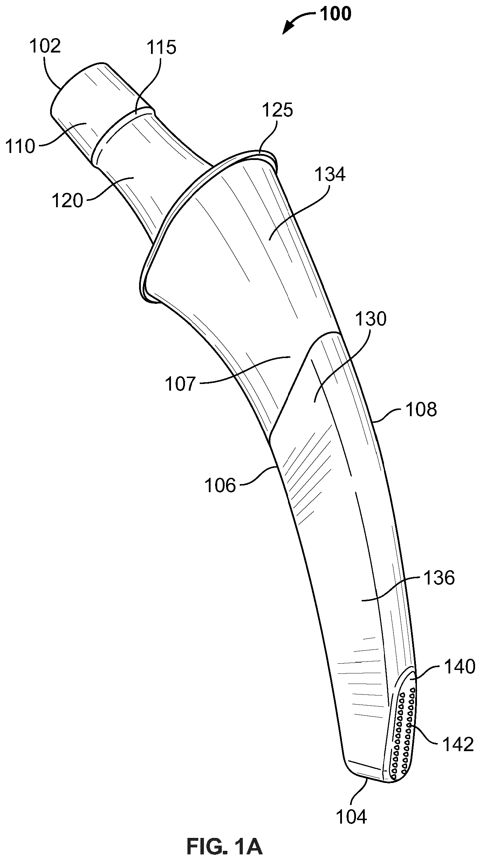

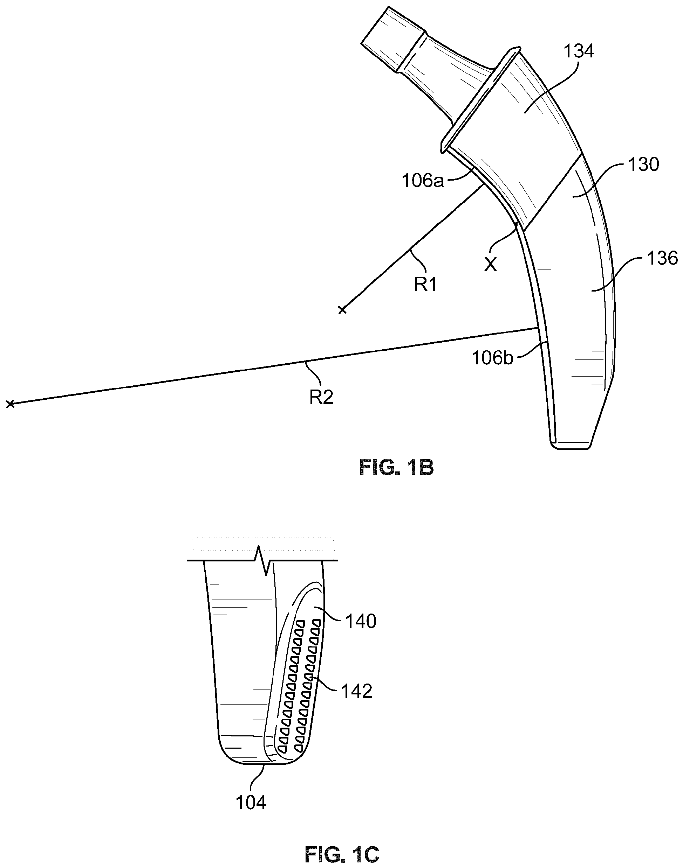

FIGS. 1-2 illustrate a hip implant 100 according to one embodiment of the disclosure. Implant 100 extends from a proximal end 102 to a distal end 104. Implant 100 may be implanted into a femur of a patient such that distal end 104 is the leading end (*i.e.,* the first end to be inserted into the patient) whereas proximal end 102 is the trailing end that remains nearer the surgeon or user during implantation. A method of implantation will be discussed below in further detail.

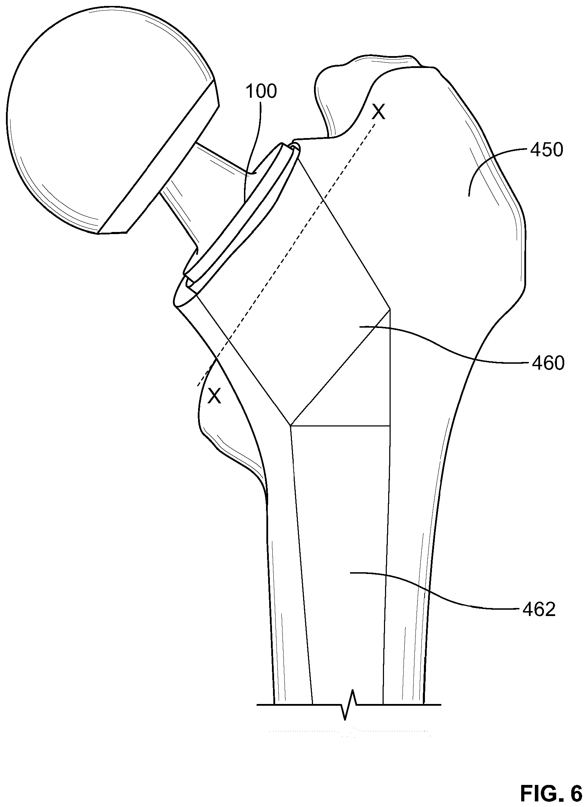

At proximal end 102, implant 100 may include a head receiving portion 110, which may be configured to receive a modular prosthetic spherical head (see FIG. 6). The spherical head may replace the anatomic femoral head and articulate with the acetabulum of the pelvis when the prosthesis is fully implanted. Head receiving portion 110 is generally frustoconical and therefore has a tapering diameter so as form a taper lock with a prosthetic head. Head receiving portion 110 has a first height. The distal end of head receiving portion 110 may be coupled to a neck 120. Neck 120 may also be frustoconical and extend distally from head receiving portion 110. Neck 120 may have a second height greater than the first height of head receiving portion 110 and a cross-sectional dimension less than the greatest cross-sectional dimension of head receiving portion 110, thus forming a peripheral lip 115 where head receiving portion 110 and neck 120 are coupled together. In some examples, the neck may have a height equal to or less than that of the head receiving portion. In still further examples, the head receiving portion may be rectangular, triangular, or any alternative shape, in which case the head may include a corresponding bore configured to fit to the shape of the head receiving portion. It is also contemplated that several parts of the implant may be modular, such as the head receiving portion, neck, proximal stem portion and/or distal stem portion.

A stem 130 may be coupled to and extend distally from the distal end of neck 120. Stem 130 may include a proximal stem portion 134 and a distal stem portion 136. The proximal end of stem 130 includes a flange 125 where stem is coupled to neck 120. Flange 125 may be generally stadium-shaped, *i.e.,* flange 125 may generally forms a rectangle with rounded corners. It is contemplated that the flange may define any shape that is suitable to prevent subsidence and does not substantially overhang the neck, such as an oval, rectangle, or the like. Flange 125 may have a length and width which are each greater than the second diameter of neck 120, forming a peripheral shoulder at the connection between neck 120 and stem 130. When implant 100 is fully implanted, flange 125 may sit at the neck resection level to provide initial stability and prevent subsidence of the stem after final seating. Stem 130 may have a medial side 106 which, when the prosthesis is implanted as intended, will face toward a medial extent of the femur and the middle of the patient's body. Stem 130 may further have a lateral side 108 which, when the prosthesis is implanted as intended, will face toward a lateral extent of the femur and the outside of the patient's body. Stem 130 may further have an anterior side 107 which will face the front of the patient's body, and a posterior side 109 which will face the rear of a patient's body. It should be noted that the anterior and posterior sides are not limited to facing in their respective directions as defined above, but are merely defined this way for ease of illustration. Implant 100 may be implanted into a first femur on one side of the patient with the anterior side facing frontward and the posterior side facing rearward. However, implant 100 may be implanted into a second femur on the opposing side of the patient with the anterior side facing rearward and the posterior side facing frontward.

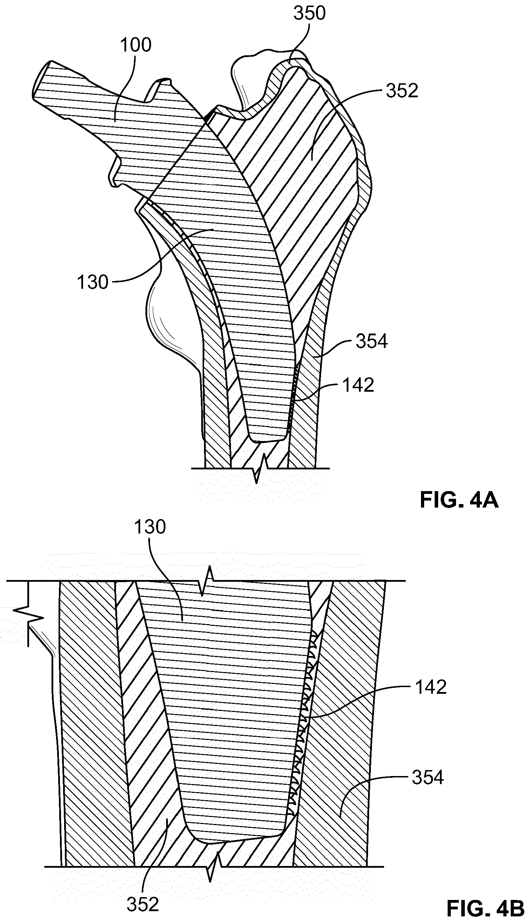

As stem 130 extends distally, stem 130 may curve toward medial side 106. Thus, medial side 106 may have a concave curvature which may transfer load to the proximal femoral neck and medial calcar region and may improve the press fit between stem 130 and the femoral neck. Lateral side 108 may have a convex curvature, which may conserve bone at the femoral neck, improve press fit, and improve stem insertion by clearing the lateral cortices within the femoral shaft. As illustrated in FIG. 1B, proximal-medial side 106a of proximal stem portion 134 may have a first radius of curvature R1 and distal-medial side 106b of distal stem portion 136 may have a second radius of curvature R2 greater than the first radius of curvature R1. Proximal-medial side 106a having first radius of curvature R1 transitions to distal-medial side 106b having second radius of curvature R2 at a transition point X. Transition point X is defined as an intersection between the curved proximal medial surface 106a and curved distal medial surface 106b. As shown, the radius of curvature R2 is greater than radius of curvature R1. In this regard the center of curvature defined at the end of R2 is located further from stem medially than the center of curvature at the end of radius R1. However, as shown, radii R1 and R2 do not intersect. Such a geometry of the stem may help to conserve bone stock in the neck region and help with the insertion of stem 130 into bone so that stem 130 conforms to a patient's cortical bone at a medial aspect of a patient's femur as it is being inserted into an intramedullary canal thereof, as depicted in FIGS. 4A-5. Further, lateral side 108 of stem 130 has a third radius of curvature also greater than the first radius of curvature R1. The curvatures of the medial and lateral sides of the implant may be modified or customized to properly fit specific patients. The curvatures may be derived from the Stryker Orthopaedic Modeling and Analytics (SOMA) database.

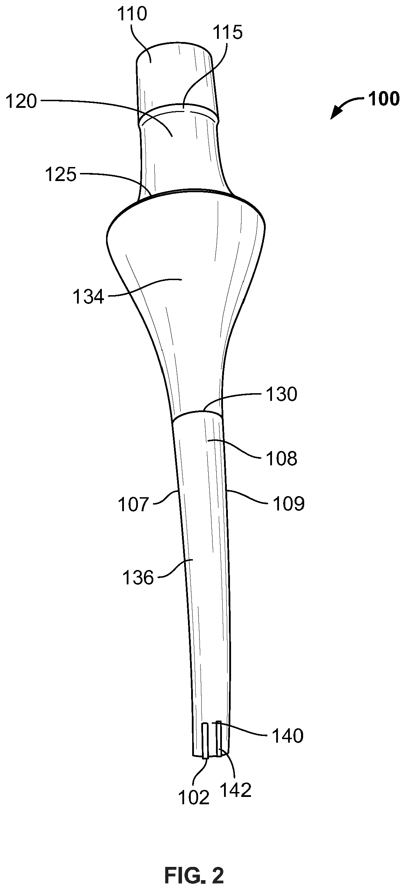

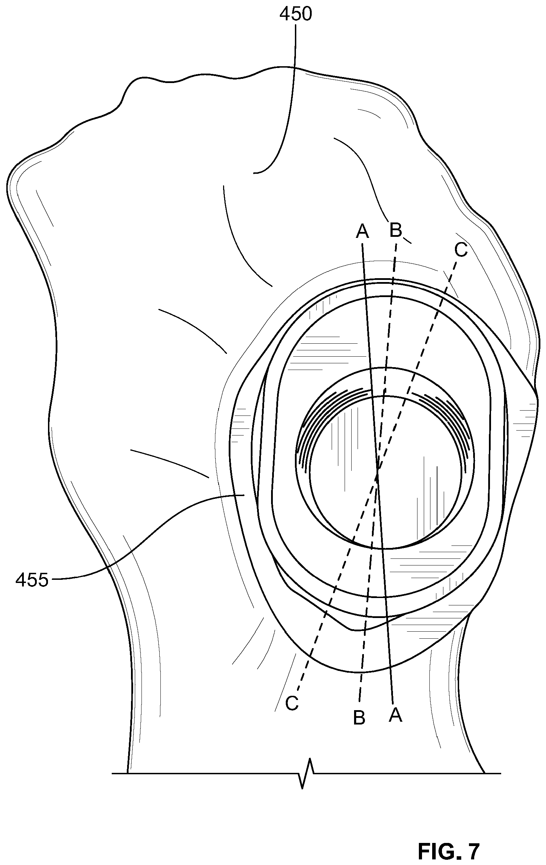

Proximal stem portion 134 may have a cross-sectional shape that is generally quadrilateral, such that the taper of proximal stem portion 134 clears all of the cortices of the femoral neck upon insertion except the general medial calcar region, which is approximately where proximal stem portion 134 is seated after insertion. As proximal stem portion 134 extends distally, the length and width of stem portion 134 may taper, wherein the length is defined by the distance between medial side 106 and lateral side 108 and the width is defined by the distance between anterior side 107 and the posterior side 109. The anterior-posterior taper is illustrated more clearly in FIG. 2. The taper of stem 130 may help to prevent unintended distal fixation and fit patients with larger mismatches in version angle between the neck and proximal femur. In other words, the taper of the stem 130 may help to insert the stem 130 evenly into the canal and help prevent anteversion or retroversion of the stem 130 in the femoral neck relative to the proximal portion of the femur. The version angle of the stem 130 is best illustrated in FIG. 7, which shows the stem 130 evenly seated within the femoral neck 455 on all sides. The tapered profile of the stem 130 may clear the distal cortices of the proximal canal to allow the stem to be fully seated onto the medial calcar region. The distal surface of the flange 125 may contact or come substantially close to contacting the medial calcar region for the femur. In the illustration of FIG. 7, the femoral neck 455, the stem 130 and the femoral canal each define a generally ovular shape, each having a major axis extending along a plane. The major axis of the femoral neck 455 is identified by axis A-A, the major axis of the stem 130 is identified by axis B-B, and the axis of the femoral canal is identified by axis C-C. As shown in FIG. 7, the major axis of each of the neck 455, stem 130 and canal extend along different planes. Thus, the tapered profile of the stem 130 may help to overcome the distinct shapes of the neck 455, stem 130 and canal shown by the varying major axes and may allow for a smooth and even insertion of the stem 130 into the femoral canal.

It is also contemplated that the entire stem 130 may taper in length and width. Proximal stem portion 134 includes a 3D porous coating to enable bone ingrowth and long-term fixation of implant 100 within the femur. A boundary between the porous coating and non-porous stem extends medially-laterally at an oblique angle relative to an axis of the stem such that the boundary is more proximal on the lateral side than on the medial side. Thus, the porous coating extends along a longer length on the medial side than on the lateral side. In this regard, the lateral side provides a longer smoother surface in order to provide clearance to enable the stem to fully seat within the canal, whereas the medial side provides greater fixation due to the additional porous surface area. The porous coating may be made from titanium or any biocompatible material such as hydroxyapatite or the like. Distal stem portion 136 is polished to avoid bony in-growth at the distal end of implant 100 and reduce stress shielding. Referring back to FIG. 1B, it should be noted that the transition point X between proximal-medial side 106a and distal-medial side 106b is located proximal to the boundary between the porous coating and the non-porous stem portion. In other words, the transition point X is located proximal to the distal-medial point of the proximal stem portion 134 having the porous coating.

As illustrated in FIG. 1A, distal stem portion 136 includes a relief surface 140 on lateral side 108. Relief surface 140 may lie in a plane which extends in a distal direction toward a central axis of stem 130 and in a proximal direction to intersect the convex curvature of lateral side 108, forming a distal end of stem 130 tapering in length and having a polygonal cross-section. In other words, the concave curvature of medial side 106 may extend from flange 125 to a distal extent of stem 130, while the convex curvature of lateral side 108 may extend from flange 125 and terminate at relief surface 140. Relief surface 140 may then extend from the convex curvature to the distal extent of stem 130. Relief surface 140 may reduce lateral impingement on the inner surface of the femur and thereby ease insertion of implant 100. Relief surface 140 includes a plurality of teeth 142 disposed along and extending from the surface.

FIG. 1C illustrates a close-up view of relief surface 140 including teeth 142. Each tooth forms a sharp prong that extends in a direction perpendicular to relief surface 140. Teeth 142 may be arranged in a grid comprising 18 rows and 3 columns. Teeth 142 may contact the lateral extent of the femur as implant 100 is being implanted, which will be discussed below in further detail with reference to the method of implantation. Teeth 142 form an abrasive surface to allow for advancement of the implant through the femoral canal. The combination of teeth 142 and relief surface 140 provide implant 100 the ability to self-broach during implantation to advance and seat itself within the femoral canal without the need for preparation of the canal with various broaches. In further examples, the teeth may be arranged on the relief surface in any number of rows and columns. In still further examples, the teeth may be arranged in an array having a plurality of rows with the teeth of every other row arranged in columns and the teeth of adjacent rows vertically offset. In still further examples, the teeth may be scattered around the relief surface in no particular arrangement positioned with varying distances between each tooth. Teeth may also be disposed on the anterior and/or posterior surfaces 107, 109 of the distal stem portion 136 such that teeth are positioned on up to three of the four surfaces of distal stem portion 136. Teeth may also be disposed on the distal tip 104 of the stem. It is contemplated that the teeth may be formed of any suitable geometry to optimize the self-broaching ability of the stem. For example, a tooth may extend straight from the surface of the stem or may have a radius of curvature. Further, a tooth may have any rake angle, break edge and may have any dimensions (*e.g.,* length, width and depth of each tooth). A tooth may be formed of a solid material all the way through or may be hollowed out.

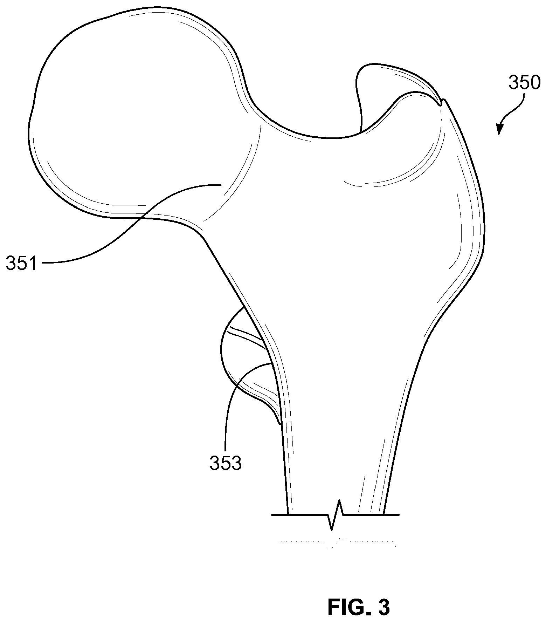

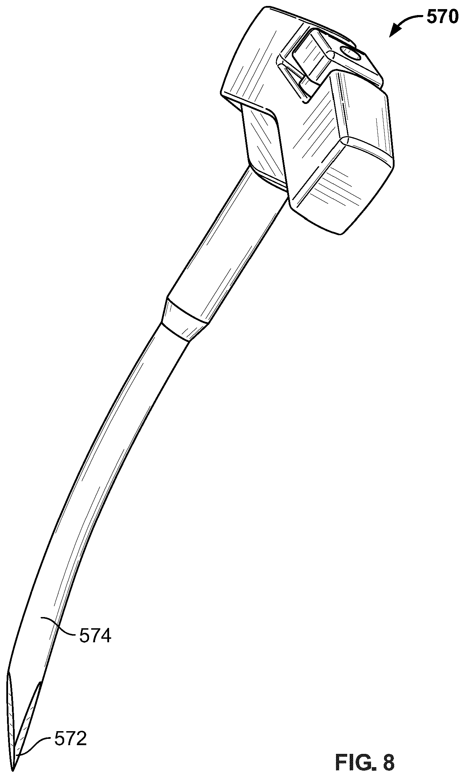

FIGS. 3-5 illustrate the resection, implantation and seating of implant 100 into a femur 350. Prior to surgery, the intended position of implant 100 may be planned using a CT-based plan of the patient. Femur 350 may be prepared robotically using a high-speed burr to resect the bone. Femur 350 may be resected through the neck 351 and the proximal canal up to the level of the lesser trochanter 353, as shown in FIG. 3. The femur may be resected such that a press fit connection may be formed between the femur and the intended fixation region of implant 100 when implanted into the femur. Robotic tracking of the burr may be employed while the burr is cutting to ensure the cut and the femur correspond to the pre-surgical plan. For some patients, *e.g*., patients with relatively smaller anatomy and/or tighter proximal canals, an instrument may be used to create a narrow pilot hole that extends into the femoral canal to prepare the canal to receive the distal portion of implant 100. The instrument used to form the pilot hole may be a curved awl, such as the instrument 570 shown in FIG. 8 having a sharp tip 572 and a curved stem 574 to form a curved hole that corresponds to the intended path of stem 130 during implantation. The instrument may be inserted past the medial boundary of the greater trochanteric canal space into the proximal femoral canal, thus creating a pilot hole through the cancellous bone of the proximal canal for the self-broaching stem. It is contemplated that the instrument may be controlled robotically or impacted manually. The instrument may be a single size that opens up a pilot hole up to a predetermined depth (*e.g.,* lesser trochanter), which may be compatible with all sizes of the self-broaching stem. It should be noted that unlike a conventional broach, the curved awl instrument may not conform to the outer profile of the stem, and is only intended to ease entry of the stem into the distal canal. In this regard, implant 100 remains self-broaching even when a curved awl is utilized to form a pilot opening. The pilot hole may ease the insertion of stem 130 into the canal, but may be narrower than stem 130 such that a degree of force may be required during implantation and implant 100 may have a snug fit within the canal after implantation. Moreover, such curved entry instrument may not have teeth on it such that the bone within the femoral canal is not resected prior to the insertion of stem 130. After the pilot hole is formed, implant 100 may be impacted into the femur. Implant 100 widens the hole as stem 130 is inserted into the pilot hole resulting in a press fit relationship between stem 130 and the bone.

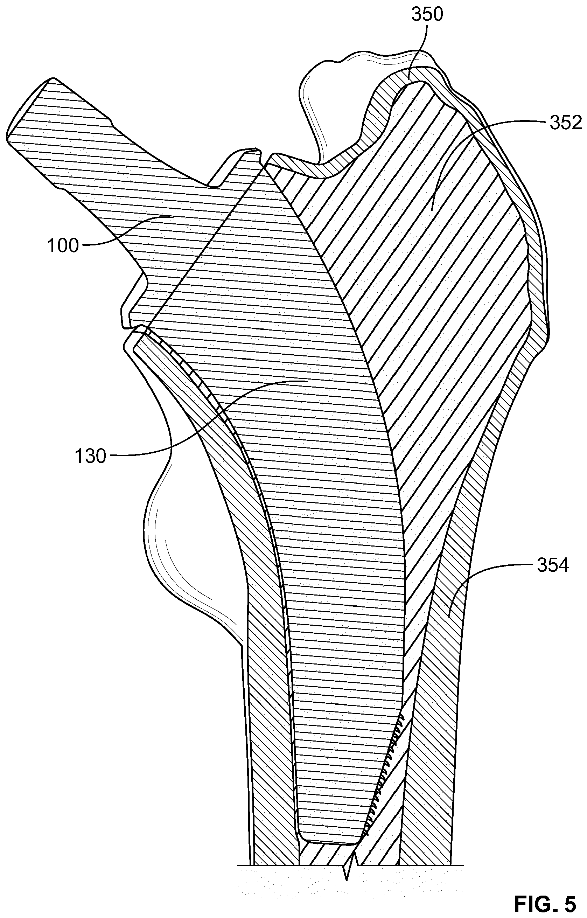

As shown in FIGS. 4A-B in which a substantial portion of stem 130 is implanted, teeth 142 disposed on relief surface 140 may be used to prepare the distal region of the femoral canal. As stem 130 is advanced distally through the cancellous bone 352 of femur 350, the distal portion of lateral side 108 may approach and contact the lateral extent of femoral cortical bone 354. This is at least due to the curved nature of stem 130 and the curved path the stem 130 takes as it is inserted into the bone. Teeth 142 and relief surface 140 allow for self-broaching of implant 100 by simultaneously preparing a pathway through the femoral canal for stem 130 while being advanced distally through the canal. FIG. 5 illustrates the final seated position of implant 100 within femur 350. Final seating of implant 100 may be achieved when flange 125 reaches the resection level, or a position that is within 2 millimeters of the resection level, and a desired press fit is achieved between the proximal portion of femur 350 and the corresponding portion of implant 100. In its final seating, medial side 106 of implant 100 abuts the medial extent of femur 350, and the length of stem 130 allows stem 130 to place its load on the medial calcar region of the proximal portion of femur 350, thus allowing for greater anatomic loading. It is also contemplated that the lateral side 108 of implant 100 may also abut a lateral extent of the femur.

It is contemplated that the pre-surgical plan of the patient may use any means of imaging, such as X-Ray, MRI, or the like, and the plan may also include 3D modeling of the implant and/or the femur of the patient through the use of CAD or similar software. It is also contemplated that any appropriate cutting means may be used to resect the femur, such as a reciprocating saw or the like. In some examples, the resection of the femur may be performed manually by a surgeon rather than robotically. It is contemplated that not all of the steps as described above are required for implantation. For example, a pilot hole may not be formed prior to implantation, but rather the implant may be impacted into the femur after the step of resecting.

FIGS. 6-7 illustrate implant 100 fully seated within a femur 450. As shown, a proximal section 460 of femur 450 that was robotically or manually resected is contrasted with a distal section 462 of femur 450 that was self-broached by the distal-lateral end of stem 130 during implantation. Additionally, axis X-X illustrates the level of resection of the femur in a conventional total hip arthroplasty in preparation for receiving an implant. Thus, the portion of femur 450 that remains intact proximal to axis X-X in FIG. 6 represents the amount of original bone that is preserved relative to a conventionally resected hip implant, thus providing each of the benefits as detailed above.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. A femoral implant (100) comprising:
a spherical head at a proximal end (102) of the implant (100);
a neck (120) extending distally from the head; and
a stem (130) extending distally from the neck (120) and having a medial side (106) adapted to face a medial extent of the femur (350) and a lateral side (108) adapted to face a lateral extent of the femur when implanted therein, the medial side (106) having a concave curvature, the lateral side (108) having a convex curvature, the stem (130) including a plurality of teeth (142) disposed on a distal portion of the lateral side (108) of the stem (130) such that the stem (130) is configured to be self-broaching when implanted into the femur, **characterized in that** the plurality of teeth (142) are disposed on a relief surface (140) located on the lateral side (108) of the stem (130), the relief surface (140) intersecting the convex curvature and extending distally therefrom.

2. The femoral implant (100) of claim 1, wherein the plurality of teeth (142) are arranged in a plurality of columns on the lateral side (108).

3. The femoral implant (100) of claim 1 or 2, wherein the stem (130) includes a flange (125) between the distal end of the neck (120) and the proximal end of the stem (130), the flange (125) having a larger cross-section than the neck (120) defining a peripheral lip (115).

4. The femoral implant (100) of any one of claims 1 to 3, wherein the proximal portion of the stem (130) includes a porous structure.

5. The femoral implant (100) of claim 4, wherein the porous structure is positioned more proximally on the lateral side (108) of the proximal stem portion (134) than the medial side (106) of the proximal stem portion (134).

6. The femoral implant (100) of claim 4 or 5, wherein the medial side (106) has a transition point (X) where the medial side (106) changes from the first radius of curvature (R1) to the second radius of curvature (R2), and the stem (130) defines a transition plane between the porous structure and a non-porous portion of the stem (130), wherein the transition point (X) is located proximal to the transition plane.

7. The femoral implant (100) of any one of claims 1 to 6, wherein the stem (130) includes an anterior side (107) adapted to face an anterior extent of the femur and a posterior side (109) adapted to face a posterior extent of the femur, the stem (130) tapering in both the medial-lateral direction and the anterior-posterior direction as the stem (130) extends distally.

8. The femoral implant (100) of any one of claims 1 to 7, wherein the relief surface (140) extends toward a central axis of the stem (130) in a distal direction.

9. The femoral implant (100) of claim 8, wherein the relief surface (140) is located at a relief portion of the stem (130), the relief portion of the stem (130) having a polygonal cross section.

10. The femoral implant (100) of any one of claims 1 to 9, wherein a distal portion of the stem (130) is polished.

11. The femoral implant (100) of any one of claims 1 to 10, wherein the concave curvature of the stem (130) extends from a proximal extent of the stem (130) to a distal extent of the stem (130).

12. The femoral implant (100) of any one of claims 1 to 11, wherein the neck (120) and stem (130) are monolithic, and the spherical head is a modular piece coupleable to the neck (120) and stem (130).

13. The femoral implant (100) of any one of claims 1 to 12, wherein the medial side (106) of the stem (130) defines a first radius of curvature (R1) on a proximal portion of the medial side (106) of the stem (130), the medial side (106) further defining a second radius of curvature (R2) on a distal portion of the medial side (106) of the stem (130).

14. The femoral implant (100) of claim 13, wherein the lateral side (108) of the stem (130) defines a third radius of curvature.

15. The femoral implant (100) of claim 14, wherein the second (R2) and third radii of curvature are different than the first radius of curvature (R1).

## Patentansprüche

1. Femurimplantat (100), umfassend:
einen kugelförmigen Kopf an einem proximalen Ende (102) des Implantats (100);
einen Hals (120), der sich distal von dem Kopf erstreckt; und
einen Schaft (130), der sich distal von dem Hals (120) erstreckt und eine mediale Seite (106), die dazu ausgebildet ist, einem medialen Ausmaß des Femurs (350) zugewandt zu sein, und eine laterale Seite (108) aufweist, die dazu ausgebildet ist, einem lateralen Ausmaß des Femurs zugewandt zu sein, wenn er darin implantiert ist, wobei die mediale Seite (106) eine konkave Krümmung aufweist, die laterale Seite (108) eine konvexe Krümmung aufweist, der Schaft (130) eine Vielzahl von Zähnen (142) umfasst, die auf einem distalen Abschnitt der lateralen Seite (108) des Schafts (130) angeordnet sind, derart dass der Schaft (130) dazu ausgestaltet ist, selbstschneidend zu sein, wenn er in den Femur implantiert wird,
**dadurch gekennzeichnet, dass** die Vielzahl von Zähnen (142) auf einer Reliefoberfläche (140) angeordnet sind, die sich auf der lateralen Seite (108) des Schafts (130) befindet, wobei die Reliefoberfläche (140) die konvexe Krümmung schneidet und sich distal davon erstreckt.

2. Femurimplantat (100) nach Anspruch 1, wobei die Vielzahl von Zähnen (142) in einer Vielzahl von Spalten auf der lateralen Seite (108) angeordnet sind.

3. Femurimplantat (100) nach Anspruch 1 oder 2, wobei der Schaft (130) einen Flansch (125) zwischen dem distalen Ende des Halses (120) und dem proximalen Ende des Schafts (130) umfasst, wobei der Flansch (125) einen größeren Querschnitt aufweist als der Hals (120), wodurch eine Umfangslippe (115) definiert wird.

4. Femurimplantat (100) nach einem der Ansprüche 1 bis 3, wobei der proximale Abschnitt des Schafts (130) eine poröse Struktur umfasst.

5. Femurimplantat (100) nach Anspruch 4, wobei die poröse Struktur proximaler an der lateralen Seite (108) des proximalen Schaftabschnitts (134) als die mediale Seite (106) des proximalen Schaftabschnitts (134) positioniert ist.

6. Femurimplantat (100) nach Anspruch 4 oder 5, wobei die mediale Seite (106) einen Übergangspunkt (X) aufweist, wo die mediale Seite (106) vom ersten Krümmungsradius (R1) zum zweiten Krümmungsradius (R2) wechselt, und der Schaft (130) eine Übergangsebene zwischen der porösen Struktur und einem nicht porösen Abschnitt des Schafts (130) definiert, wobei der Übergangspunkt (X) sich proximal zur Übergangsebene befindet.

7. Femurimplantat (100) nach einem der Ansprüche 1 bis 6, wobei der Schaft (130) eine vordere Seite (107), die dazu ausgebildet ist, einem vorderen Ausmaß des Femurs zugewandt zu sein, und eine hintere Seite (109) umfasst, die dazu ausgebildet ist, einem hinteren Ausmaß des Femurs zugewandt zu sein, wobei der Schaft (130) sich in sowohl der medial-lateralen Richtung als auch der Vorn-Hinten-Richtung verjüngt, während der Schaft (130) sich distal erstreckt.

8. Femurimplantat (100) nach einem der Ansprüche 1 bis 7, wobei die Reliefoberfläche (140) sich hin zu einer Mittelachse des Schafts (130) in einer distalen Richtung erstreckt.

9. Femurimplantat (100) nach Anspruch 8, wobei die Reliefoberfläche (140) sich an einem Reliefabschnitt des Schafts (130) befindet, wobei der Reliefabschnitt des Schafts (130) einen vieleckigen Querschnitt aufweist.

10. Femurimplantat (100) nach einem der Ansprüche 1 bis 9, wobei ein distaler Abschnitt des Schafts (130) poliert ist.

11. Femurimplantat (100) nach einem der Ansprüche 1 bis 10, wobei die konkave Krümmung des Schafts (130) sich von einem proximalen Ausmaß des Schafts (130) zu einem distalen Ausmaß des Schafts (130) erstreckt.

12. Femurimplantat (100) nach einem der Ansprüche 1 bis 11, wobei der Hals (120) und der Schaft (130) monolithisch sind und der kugelförmige Kopf ein modulares Teil ist, das an den Hals (120) und den Schaft (130) koppelbar ist.

13. Femurimplantat (100) nach einem der Ansprüche 1 bis 12, wobei die mediale Seite (106) des Schafts (130) einen ersten Krümmungsradius (R1) auf einem proximalen Abschnitt der medialen Seite (106) des Schafts (130) definiert, wobei die mediale Seite (106) ferner einen zweiten Krümmungsradius (R2) auf einem distalen Abschnitt der medialen Seite (106) des Schafts (130) definiert.

14. Femurimplantat (100) nach Anspruch 13, wobei die laterale Seite (108) des Schafts (130) einen dritten Krümmungsradius definiert.

15. Femurimplantat (100) nach Anspruch 14, wobei der zweite (R2) und der dritte Krümmungsradius sich von dem ersten Krümmungsradius (R1) unterscheiden.

## Revendications

1. Implant fémoral (100) comprenant :
une tête sphérique à une extrémité proximale (102) de l'implant (100) ;
un col (120) s'étendant distalement à partir de la tête ; et
une tige (130) s'étendant distalement à partir du col (120) et comportant un côté médial (106) adapté pour faire face à une partie médiale du fémur (350) et un côté latéral (108) adapté pour faire face à une partie latérale du fémur lorsqu'il est implanté dans celui-ci, le côté médial (106) présentant une courbure concave, le côté latéral (108) ayant une courbure convexe, la tige (130) comprenant une pluralité de dents (142) disposées sur une partie distale du côté latéral (108) de la tige (130) de telle sorte que la tige (130) est configurée pour s'auto-aléser lorsqu'elle est implantée dans le fémur,
**caractérisé en ce que** la pluralité de dents (142) est disposée sur une surface en relief (140) située sur le côté latéral (108) de la tige (130), la surface en relief (140) coupant la courbure convexe et s'étendant distalement à partir de celle-ci.

2. Implant fémoral (100) selon la revendication 1, dans lequel la pluralité de dents (142) est disposée en une pluralité de colonnes sur le côté latéral (108).

3. Implant fémoral (100) selon la revendication 1 ou 2, dans lequel la tige (130) comprend une bride (125) entre l'extrémité distale du col (120) et l'extrémité proximale de la tige (130), la bride (125) ayant une section transversale plus grande que le col (120) définissant une lèvre périphérique (115).

4. Implant fémoral (100) selon l'une quelconque des revendications 1 à 3, dans lequel la partie proximale de la tige (130) comprend une structure poreuse.

5. Implant fémoral (100) selon la revendication 4, dans lequel la structure poreuse est positionnée plus proximalement sur le côté latéral (108) de la partie proximale de la tige (134) que sur le côté médial (106) de la partie proximale de la tige (134).

6. L'implant fémoral (100) selon la revendication 4 ou 5, dans lequel le côté médial (106) comporte un point de transition (X) où le côté médial (106) passe du premier rayon de courbure (R1) au deuxième rayon de courbure (R2), et la tige (130) définit un plan de transition entre la structure poreuse et une partie non poreuse de la tige (130), dans lequel le point de transition (X) est situé à proximité du plan de transition.

7. Implant fémoral (100) selon l'une quelconque des revendications 1 à 6, dans lequel la tige (130) comprend un côté antérieur (107) adapté pour faire face à une extension antérieure du fémur et un côté postérieur (109) adapté pour faire face à une extension postérieure du fémur, la tige (130) se rétrécissant à la fois dans la direction médiale-latérale et dans la direction antéro-postérieure à mesure que la tige (130) s'étend distalement.

8. Implant fémoral (100) selon l'une quelconque des revendications 1 à 7, dans lequel la surface de relief (140) s'étend vers un axe central de la tige (130) dans une direction distale.

9. Implant fémoral (100) selon la revendication 8, dans lequel la surface de relief (140) est située au niveau d'une partie de relief de la tige (130), la partie de relief de la tige (130) ayant une section transversale polygonale.

10. Implant fémoral (100) selon l'une quelconque des revendications 1 à 9, dans lequel une partie distale de la tige (130) est polie.

11. Implant fémoral (100) selon l'une quelconque des revendications 1 à 10, dans lequel la courbure concave de la tige (130) s'étend depuis une extrémité proximale de la tige (130) jusqu'à une extrémité distale de la tige (130).

12. Implant fémoral (100) selon l'une quelconque des revendications 1 à 11, dans lequel le col (120) et la tige (130) sont monolithiques, et la tête sphérique est une pièce modulaire pouvant être couplée au col (120) et à la tige (130).

13. Implant fémoral (100) selon l'une quelconque des revendications 1 à 12, dans lequel le côté médial (106) de la tige (130) définit un premier rayon de courbure (R1) sur une partie proximale du côté médial (106) de la tige (130), le côté médial (106) définissant en outre un deuxième rayon de courbure (R2) sur une partie distale du côté médial (106) de la tige (130).

14. Implant fémoral (100) selon la revendication 13, dans lequel le côté latéral (108) de la tige (130) définit un troisième rayon de courbure.

15. Implant fémoral (100) selon la revendication 14, dans lequel les deuxième (R2) et troisième rayons de courbure sont différents du premier rayon de courbure (R1).
